# EUROPEAN PATENT APPLICATION

(11) **EP 2 719 412 A1**
(43) Date of publication of application: **16.04.2014**
(21) Application number: 12188073.6
(22) Date of filing: 11.10.2012
(51) Int. Cl.: A61M 5/168, G01F 11/28, G01F 23/26

(54) **Precision liquid dispensing device**

(71) Applicant: IN. Medica, d.o.o., 8310 Sentjernej (SI)
(72) Inventor: Pavlin, Marko, 8000 Novo Mesto (SI)
(74) Representative: Ivancic, Bojan

(57) **Abstract**

The present invention refers to a precision liquid dispensing device, in particular to a portable disposable device for controlled medication dispensing during certain time period, such as insulin for instance and similar. It is provided for according to the present invention that said device comprises a flow conduit (2) resembling in the plane of a flat shaped substrate (1) a meander like shape in the form of waves (3), a conductive sensor means (6, 7, 8) being placed on said conduit (2) in a rectangular grid, said conductive sensor means (6, 7, 8) crosses the course of said conduit in at least one point. Said conductive sensor means (6, 7, 8) are connected to a control unit (10).

## Description

The present invention refers to a precision liquid dispensing device, in particular to a portable disposable device for controlled medication dispensing during certain time period, such as insulin for instance and similar.

Devices as mentioned in the foregoing are known and comprise, in general, a longitudinal cylindrical container wherefrom liquid, such as insulin for instance, being dispensed by means of a piston inserted into said container. A drawback of the known devices is represented by a relative large length thereof, since initially, with the filled container, said piston with a piston rod is retracted from the container which in turn represents a double length of the device. Due to this fact said known devices are rather unhandy and user-unfriendly.

It is the object of the present invention to create a precision liquid dispensing device, in particular to a portable disposable device for controlled medication dispensing during certain time period, such as insulin for instance and similar, which remedies drawbacks of the known solutions.

The object as set above is solved by characteristics set forth in a characterising clause of the claim 1.

The invention is further described in detail by way of non-limiting preferred embodiment, and with a reference to the accompanying drawing, where
- Fig. 1: shows a three-dimensional view of a device according to the invention,
- Fig. 2: shows a schematic view of a device according to the invention.

A precision liquid dispensing device according to the invention is intended for use with the flat shaped substrate 1 such as skin patch. To this end, said device is formed of a relatively small diameter flow conduit 2 resembling in the plane of said substrate 1 a meander like shape, preferably in the form of regular waveforms 3, adhered to said substrate 1. Optionally, both the conduit 2 and the substrate 1 may be made of transparent material enabling quick insight into the condition of the liquid within said conduit 2. A conductive sensor means 6 is placed on said conduit 2 in the area of a crest 4 and in the area of a through 5 of each wave 3, said sensor means 6 crosses the course of the conduit 2 in at least one point. In addition, at least one additional conductive sensor means 7 is arranged between said crest 4 and said through 5 of each wave 3, said sensor means 7 being placed on said conduit 2 and crossing the course of the conduit 2 in at least one point. In essence, the additional conductive sensor means 7 is arranged approximately perpendicular to the course of the conduit 2. Furthermore, at least one additional conductive sensor means 8 is arranged at the inlet section 2a and at the outlet section 2b of the conduit 2 said sensor means 8 being placed on said conduit 2 and crossing the course of the conduit 2 in at least one point. Said conductive sensor means 6, 7, 8 are connected by means of lines 9 to a control unit 10. A pumping means 11 is also connected to said control unit 10, whereby said pumping means sucks liquid to be dispensed via a suction conduit attached to the outlet section 2b of the conduit 2 from a device according to the invention and forwards it to the user.

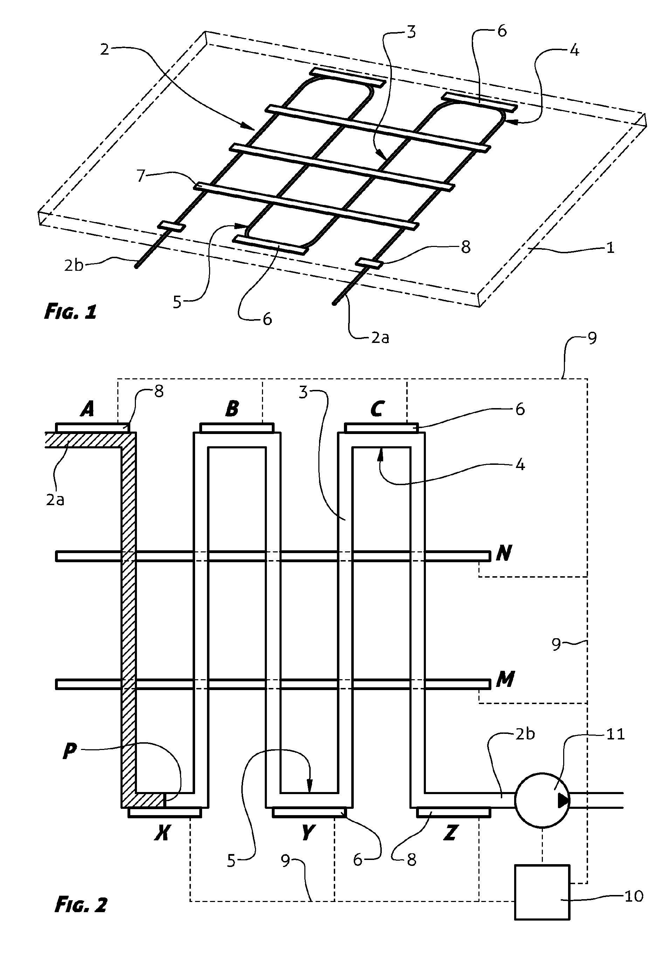

The course of both said conduit 2 and said conductive means 6, 7, 8 creates in the manner as described above a rectangular grid, i.e. a raster, which enables to accurately and precisely determine a current position of a liquid head in said conduit 2 and, as a result, the amount of the liquid to be dispensed already drawn out. Namely, the position of said liquid head is clearly determined by an intersection of said conduit 2 and each conductive means 6, 7, 8 intersecting said conduit 2. In essence, with the device according to the invention changes of dielectric properties of the substances between said conductive means 6, 7, 8 are detected, i.e. the presence or the absence of the liquid at the certain point in the conduit 2. Figure 2, for example, shows the position of the liquid head P flowing in from the inlet section 2a into said conduit 2, wherein electromagnetic interaction takes place between the liquid in the conduit 2 and each conductive means 6, 7, 8 already passed by the liquid in the conduit 2. In the embodiment, as shown, the interaction takes place, in particular the impedance interaction in the direction *A-N, A-M* and *X-M.* Said impedance interactions are transmitted to said control unit 10 which, on the basis of said information, calculates the actual position of the head P of the liquid to be dispensed and, respectively, the amount of the liquid flowing through said conduit 2 in a certain time period. Obviously, said accuracy of determination of the current position of the liquid head *P* depends on density of said raster, in particular on the number of said conductive means 6, 7, 8 arranged in the area between the crest 4 and the through 5 of each wave 3.

When in turn liquid flows through said flow conduit 2 which is not suitable for the impedance interaction with said conductive means 6, 7, 8, such as for example an active substance where a weak current through the liquid would substantially influence the characteristics thereof, or an aggressive liquid which would damage the electric conductive lines, a capacitive or inductive principle of detection can be used as said electromagnetic interaction. In such a case, all conductive lines are separated from the liquid in said conduit 2 by means of a dielectric layer, such as a plastic film for instance.

The amount of the drawn out liquid to be dispensed is measured on said pumping means 11 in a manner known per se. In addition, the amount of the drawn out liquid to be dispensed is measured by means of said raster formed by said conduit 2 and said conductive means 6, 7, 8. As already mentioned, said conductive means detects each position of the head of the liquid to be dispensed upon which the device according to the invention calculates the amount of the liquid to be dispensed remaining in the conduit 2 and, respectively, the amount of the liquid already pumped out. Comparing the dispensed amount of the liquid obtained directly at the pumping means 11 with the amount of the liquid obtained by means of said conductive means 6, 7, 8, it can be very simple to determine the exact amount of the pumped-out liquid to be dispensed. In such a manner, it can be efficiently and reliably prevented to overdose and under-dose the liquid to be dispensed by means of the device according to the present invention

The embodiment represented in the drawing provides for the use of the conductive means 6, 7, 8 of different length. However, it is obvious for the person skilled in the art that other structures are possible, such as the same length of the conductive means 6, 7, 8, wireless connection of said conductive means 6, 7, 8 with the control unit 10 and similar, without departing from the spirit and scope of the invention.

## Claims

1. A precision liquid dispensing device, in particular a portable disposable device for controlled medication dispensing during certain time period, such as insulin for instance and similar, ***characterized in that*** it comprises a flow conduit (2) resembling in the plane of a flat shaped substrate (1) a meander like shape in the form of waves (3), a conductive sensor means (6, 7, 8) being placed on said conduit (2) in a rectangular grid, said conductive sensor means (6, 7, 8) crosses the course of said conduit in at least one point, whereby said conductive sensor means (6, 7, 8) are connected to a control unit (10).

2. A device according to claim 1, ***characterized in that*** the position of a head (P) of the liquid flowing from an inlet section (2a) into said conduit (2) is determined by means of electromagnetic interaction between the liquid in the conduit (2) and each conductive sensor means (6, 7, 8) being passed by the liquid in the conduit (2).

3. A device according to claim 1, ***characterized in that*** the conductive sensor means (6) is arranged on said conduit (2) in the area of a crest (4) and in the area of a through (5) of each wave (3).

4. A device according to claim 1, ***characterized in that*** at least one additional conductive sensor means (8) is arranged at the inlet section (2a) and at the outlet section (2b) of the conduit (2).

5. A device according to claims 1 and 2, ***characterized in that*** said electromagnetic interaction is provided as an impedance, capacitive or inductive interaction.

6. A device according to any of the preceding claim, ***characterized in that*** at least one additional conductive sensor means (7) is arranged between said crest (4) and said through (5) of each wave (3).

7. A device according to any of the preceding claim, ***characterized in that*** a pumping means (11) is connected to said control unit (10) in order to transport the liquid to be dispensed via a suction conduit attached to the outlet section (2b) of said conduit (2).
